(19) Europäisches Patentamt
European Patent Office
Office européen des brevets

(11) EP 2 410 002 A1

(12) **EUROPEAN PATENT APPLICATION**
published in accordance with Art. 153(4) EPC

(43) Date of publication:
**25.01.2012 Bulletin 2012/04**

(21) Application number: **10753591.6**

(22) Date of filing: **15.03.2010**

(51) Int Cl.:
***C08G 61/00*** *(2006.01)* ***H01L 51/30*** *(2006.01)*
***H01L 51/42*** *(2006.01)*

(86) International application number:
**PCT/JP2010/054735**

(87) International publication number:
**WO 2010/107100 (23.09.2010 Gazette 2010/38)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **17.03.2009 JP 2009063978**

(71) Applicant: **Sumitomo Chemical Company, Limited
Tokyo 104-8260 (JP)**

(72) Inventors:
- **YOSHIMURA, Ken
Tsukuba-shi
Ibaraki 305-0821 (JP)**
- **KATO, Takehito
Oyama-shi
Tochigi 323-0811 (JP)**

(74) Representative: **Vossius & Partner
Siebertstrasse 4
81675 München (DE)**

(54) **COMPOUND AND ELEMENT USING SAME**

(57) A compound which contains a structure represented by formula (1) and has a light absorption end wavelength of 600 nm or more.

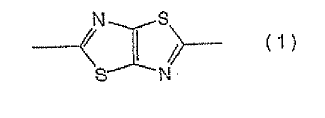

(1)

EP 2 410 002 A1

## Description

TECHNICAL FIELD

The present invention relates to a compound having a specific structure, and a device using the same.

BACKGROUND ART

In order to prevent global warming, there has recently been required to reduce $CO_2$ emitted into the atmosphere. Therefore, for example, there is made a proposal of the employment of a solar system using a p-n junction silicon-based solar battery or the like. However, high temperature and high vacuum conditions are required in the production process of single crystal, polycrystal and amorphous silicon as materials of the silicon-based solar battery.

On the other hand, an organic thin film solar battery such as an organic solar battery would be able to be produced at a low price by only an applying process without a high temperature and high vacuum process, and thus it has attracted special interest recently. For example, there is known an organic solar battery containing a composition of a copolymer composed of a repeating unit (A) and a repeating unit (B) (Applied Physics Letters Vol. 84, No. 10, 1653-1655 (2004)).

$C_8H_{17}$ $C_8H_{17}$

Repeating unit (A) Repeating unit (B)

DISCLOSURE OF THE INVENTION

However, a photoelectric conversion device such as an organic solar battery produced using the above copolymer does not necessarily exhibit sufficient conversion efficiency.

An object of the present invention is to provide a compound which can give a device having excellent performances when used as a material of a device such as a photoelectric conversion device.

First, the present invention provides a compound which contains a structure represented by formula (1) and has a light absorption end wavelength of 600 nm or more.

(1)

Second, the present invention provides a device comprising a first electrode and a second electrode, and an active layer between the first electrode and the second electrode, the active layer containing the above compound.

Third, the present invention provides a method for producing a device comprising a first electrode and a second electrode, and an active layer between the first electrode and the second electrode, the method including steps of applying a solution containing the above compound and a solvent on the first electrode by a applying method to form the active layer, and forming a second electrode on the active layer.

MODE FOR CARRYING OUT THE INVENTION

The present invention will be described in detail below.

The present invention relates to a compound which contains a structure represented by formula (1) and has a light absorption end wavelength of 600 nm or more.

(1)

The structure represented by formula (1) is a divalent group represented by formula (1). Two bonds of this group respectively combine with an atom such as a hydrogen atom or a halogen atom, a structure represented by formula (1), a structure different from the structure represented by formula (1) and the like.

The compound of the present invention may be a compound containing one structure represented by formula (1), or may be a compound containing a plurality of structures represented by formula (1).

The compound of the present invention may be a low-molecular compound, or may be a polymer compound.

The compound of the present invention may include a structure different from the structure represented by formula (1).

Examples of the structure different from the structure represented by formula (1) include a monovalent aromatic group, a divalent aromatic group, a trivalent aromatic group and the like.

The total of the formula weight of the structure represented by formula (1) is usually from 0.0001 to 0.5, preferably from 0.0005 to 0.4, and more preferably from 0.001 to 0.3, based on the molecular weight of the compound of the present invention. When the compound of the present invention is a polymer compound, the molecular weight is a polystyrene-equivalent number average molecular weight.

When the compound of the present invention is used in a photoelectric conversion device, the compound of the present invention preferably has a light absorption end wavelength of 650 nm or more, more preferably 700 nm or more, and still more preferably 720 nm or more, from the viewpoint of conversion efficiency of the photoelectric conversion device.

In the compound of the present invention, the structures represented by formula (1) may combine with each other directly, or through a conjugate-forming unit, the structures different from the structure represented by formula (1) may combine with each other directly, or through a conjugate-forming unit, and the structure different from the structure represented by formula (1) may combine with the structure represented formula (1) directly, or through the other conjugate-forming unit.

The conjugate in the present invention refers to a state where an unsaturated bond, a single bond and an unsaturated bond are associated in this order and two $\pi$ bonds of a $\pi$-orbital are next to each other, and the respective $\pi$-electrons are arranged in parallel and are not localized on a double bond or a triple bond, but spread over a neighboring single bond and are delocalized. As used herein, the above unsaturated bond mean a double bond or a triple bond.

Examples of the conjugate-forming unit include an alkenylene group and an alkynylene group. Examples of the alkenylene include a vinylene group, a propynylene group, a butenylene group, a stynylene group and the like. Examples of the alkynylene group include an ethynylene group.

The structure different from the structure represented by formula (1) is preferably a divalent aromatic group. Examples of the divalent aromatic group include groups represented by formulas 8 to 131.

R R
R
R R
R 8

9

10

11

12 13 14 15

16 17 18 19

20 21 22

23 24 25

26

27

28

29

30

31

32

33

34

35

R R R R R R R R 36

R R R R R R R R 37

R R R R R R R R 38

R R R R R R R R R R 39

R R R R R R R R R R 40

R R R R R R R R R R 41

R R R R R R R R R R 42

R R R R R R R R R R R R R R R R 43

R R R R R R R R R R R R R R R R 44

45 46 47 48 49

50 51 52 53 54

55 56 57 58 59

60 61 62 63 64

65 66 67 68 69

70 71 72 73 74

75 76 77 78

79 80 81

82 83 84

85 86 87

R R R R Si 88 R R; R R N 89 R; R R O 90; R R S 91; R R Se 92

R R R N R 93 R R; R R O R R 94; R R Se H H 95

R R R N N 96 R; R R O N 97 R; R R S N 98 R; R R Se N 99 R

R R R R Si R R 100; R R R R Si N R 101; R R N S N 102; R R N O N 103

R R S S 104 R R; R R S S S R 105 R R R; R R R R R R S R R R R 106

107 108 109

110 111 112

113 114

115 116

117

118 119

120 121

122 123

124 125

126 127

128 129 130

R R R R R R R R R R R R

131

In formulas 8 to 131, R represents a hydrogen atom or a substituent. A plurality of R's may be the same or different. When R is a substituent, examples of the substituent include an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an amide group, a monovalent heterocyclic group, a carboxyl group, a substituted carboxyl group, a nitro group, and a cyano group. The hydrogen atom included in these substituents may be substituted with a fluorine atom. $Y_5$'s may be the same or different and represent a nitrogen atom or =CH-. $Y_5$ is preferably a nitrogen atom.

As the alkyl group, a group having 1 to 30 carbon atoms is usually used, and examples thereof include chain alkyl groups such as a methyl group, an ethyl group, a n-propyl group, an isopropyl group, a n-butyl group, an isobutyl group, a sec-butyl group, a t-butyl group, a n-pentyl group, an isopentyl group, a 2-methylbutyl group, a 1-methylbutyl group, a n-hexyl group, an isohexyl group, a 3-methylpentyl group, a 2-methylpentyl group, a 1-methylpentyl group, a heptyl group, an octyl group, an isooctyl group, a 2-ethylhexyl group, a nonyl group, a decyl group, an undecyl group, a dodecyl group, a tetradecyl group, a hexadecyl group, an octadecyl group and an eicosyl group; and cycloalkyl groups such as a cyclopentyl group, a cyclohexyl group and an adamantyl group.

As the alkoxy group, a group having 1 to 30 carbon atoms is usually used, and examples thereof include a methoxy group, an ethoxy group, a propoxy group, an isopropoxy group, a butoxy group, a cyclopentyloxy group a cyclohexyloxy group and the like.

As the alkylthio group, a group having 1 to 24 carbon atoms is usually used, and examples thereof include a methylthio group, an ethylthio group, a propylthio group, an octylthio group, a decylthio group, a dodecylthio group, a tetradecylthio group, a hexadecylthio group, an octadecylthio group, an eicosylthio group, a docosylthio group, and a tetracosylthio group.

As the aryl group, a group having 6 to 20 carbon atoms is usually used, and examples thereof include a phenyl group, a naphthyl group, an anthryl group, and a pyrene group.

As the aryloxy group, a group having 6 to 20 carbon atoms is usually used, and examples thereof include a phenoxy group, and a naphthoxy group.

As the arylthio group, a group having 6 to 20 carbon atoms is usually used, and examples thereof include a phenylthio group, and a naphthylthio group.

As the arylalkyl group, a group having 7 to 20 carbon atoms is usually used, and examples thereof include a benzyl group, and a phenethyl group.

As the arylalkoxy group, a group having 7 to 20 carbon atoms is usually used, and examples thereof include a phenoxy group, an o-, m- or p-tolyloxy group, a 1- or 2-naphthoxy group, and an anthroxy group.

As the arylalkylthio group, a group having 7 to 20 carbon atoms is usually used, and examples thereof include a benzylthio group, and a phenylethylthio group.

As the arylalkenyl group, a group having 8 to 20 carbon atoms is usually used, and examples thereof include a styryl group.

As the arylalkynyl group, a group having 8 to 20 carbon atoms is usually used, and examples thereof include a phenylacetylenyl group.

Examples of the substituted amino group ($-NQ^1_2$ ($Q^1$ represents a substituent such as an alkyl group, an aryl group or a monovalent heterocyclic group, or a hydrogen atom, and at least one of $Q^1$ is a substituent) include a monoalkylamino group, a dialkylamino group, a monoarylamino group, and a diarylamino group. Examples of the alkyl group and the aryl group contained in the substituted amino group include the same groups as those described above. The substituted amino group usually has 1 to 10 carbon atoms.

Examples of the substituted silyl group ($-SiQ^2_3$ ($Q^2$ represents a substituent such as an alkyl group, an aryl

group or a monovalent heterocyclic group, or a hydrogen atom, and at least one of $Q^2$ is a substituent) include a silyl group substituted with an alkyl group, an aryl group and the like. Examples of the alkyl group and the aryl group contained in the substituted silyl group include the same groups as those described above. The substituted silyl group usually has 1 to 18 carbon atoms.

Examples of the halogen atom include a fluorine atom, a chlorine atom, a bromine atom and an iodine atom.

As the acyl group, a group having 1 to 20 carbon atoms is usually used, and examples thereof include a formyl group, an acetyl group, a propanoyl group, and a butanoyl group.

As the acyloxy group, a group having 2 to 20 carbon atoms is usually used, and examples thereof include a butyryloxyethyl group.

It is possible to use, as the amide group, a non-substituted amide group represented by $-CONH_2$, an amide group represented by $-CON(R^{500})_2$ ($R^{500}$ represents an alkyl group or an aryl group, and two $R^{500}$'s may be the same or may be different with each other) substituted with an alkyl group or an aryl group and the like. Examples of the alkyl group and the aryl group contained in the amide group include the same groups as those described above. The amino group usually has 1 to 18 carbon atoms.

Examples of the monovalent heterocyclic group include a group in which one hydrogen atom has been removed from a heterocyclic compound. The monovalent heterocyclic group usually has 4 to 20 carbon atoms.

Examples of the heterocyclic compound include furan, thiophene, pyrrole, pyrroline, pyrrolidine, oxazole, isoxazole, thiazole, isothiazole, imidazole, imidazoline, imidazolidine, pyrazole, pyrazoline, pyrazolidine, furazan, triazole, thiadiazole, oxadiazole, tetrazole, pyran, pyridine, piperidine, thiopyran, pyridazine, pyrimidine, pyrazine, piperazine, morpholine, triazine, benzofuran, isobenzofuran, benzothiophene, indole, isoindole, indolizine, indoline, isoindoline, chromene, chroman, isochroman, benzopyran, quinoline, isoquinoline, quinolizine, benzoimidazole, benzothiazole, indazole, naphthyridine, quinoxaline, quinazoline, quinazolidine, cinnoline, phthalazine, purine, pteridine, carbazole, xanthene, phenanthridine, acridine, β-carboline, pyrimidine, phenanthroline, thianthrene, phenoxathiin, phenoxazine, phenothiazine and phenazine. The preferable monovalent heterocyclic group is a monovalent aromatic heterocyclic group.

As the substituted carboxyl group (a group represented by $-COOQ^3$ ($Q^3$ represents a substituent such as an alkyl group, an aryl group, or a monovalent heterocyclic group)), a group having 2 to 20 carbon atoms is usually used, and examples thereof include a methoxycarbonyl group (methyl ester), an ethoxycarbonyl group (ethyl ester), and a butoxycarbonyl group (a butyl ester).

a and b are the same or different and represent the number of repeating units, and are usually from 1 to 5, preferably from 1 to 3, and more preferably 1.

Among the divalent aromatic group, a divalent aromatic group in which two hydrogen atoms have been removed from an aromatic fused ring formed by the condensation of two or more aromatic rings selected from the group consisting of an aromatic 7-membered ring, an aromatic 6-membered ring and an aromatic 5-membered ring is preferred, from the viewpoint of an increase in light absorption end wavelength of the present invention. Among the divalent aromatic group, a divalent aromatic group in which the number of the atoms constituting the aromatic fused ring existing in the shortest path between two atoms having a bond is 3 or 4, including atoms having a bond site is more preferred.

Examples of the "divalent aromatic group in which the number of the atoms constituting the aromatic fused ring existing in the shortest path between two atoms having a bond is 3 or 4, including atoms having a bond site" include groups represented by formulas (A) to (C). Rings H to K in formulas are expressed by only a σ bond by omitting π bonds among all bonds of the rings.

X50
C
C
H
(A)
R50
R51
C
C
C
C
I
(B)
J
C
C
C
C
K
(C)

In formulas (A) to (C), rings H and I are the same or different and represent a polycyclic aromatic ring composed of two or more 5- to 7-membered rings and may have a substituent; rings J and K are the same or different and represent

a monocyclic aromatic ring or a polycyclic aromatic ring; $X_{50}$ represents a sulfur atom, an oxygen atom, a selenium atom or -N($R_{60}$)-; $R_{60}$'s are the same or different and represent a hydrogen atom or a substituent; and $R_{50}$ and $R_{51}$ are the same or different and represent a hydrogen atom or a substituent, and $R_{50}$ and $R_{51}$ may combine to form a cyclic structure.

The shortest path between two atoms having a bond will be described.

For example, in case of formula (A), atoms having a bond are carbon atoms 1 and 3 shown below. The shortest path is 1-2-3. Therefore, the number of atoms constituting the aromatic fused ring existing in the shortest path between two atoms having a bond is 3 including atoms having a bond (bonding site).

②
X50
①
③
C
C
H

. (A)

In case of formula (B), atoms having a bond are carbon atoms 1 and 4 shown below. The shortest path is 1-2-3-4. Therefore, the number of atoms constituting the aromatic fused ring existing in the shortest path between two atoms having a bond is 4 including atoms having a bond (bonding site).

R50
②
R51
③
C
C
①
④
C
C
I

. (B)

In case of formula (C), atoms having a bond are carbon atoms 1 and 4 shown below. The shortest path is 1-2-3-4. Therefore, the number of atoms constituting the aromatic fused ring existing in the shortest path between two atoms having a bond is 4 including atoms having a bonding site.

J
④
C
②
③
C
C
①
C
K

. (C)

When rings J and K are monocyclic aromatic rings, examples of the monocyclic aromatic ring include a benzene ring, a pyrrole ring, a furan ring, a thiophene ring, an oxazole ring, a thiazole ring, a thiadiazole ring, a pyrazole ring, a pyridine ring, a pyrazine ring, an imidazole ring, a triazole ring, an isoxazole ring, an isothiazole ring, a pyrimidine ring, a pyridazine ring, and a triazine ring.

When rings J and K are polycyclic aromatic rings, examples of the polycyclic aromatic ring include an aromatic ring formed by the condensation of the monocyclic aromatic ring with any ring. Examples of the ring condensed to the monocyclic aromatic ring include a furan ring, a thiophene ring, a pyrrole ring, a pyrroline ring, a pyrrolidine ring, an oxazole ring, an isoxazole ring, a thiazole ring, an isothiazole ring, a thiadiazole ring, an imidazole ring, an imidazoline ring, an imidazolidine ring, a pyrazole ring, a pyrazoline ring, a pyrazolidine ring, a furazan ring, a triazole ring, a thiadiazole ring, an oxadiazole ring, a tetrazole ring, a pyran ring, a pyridine ring, a piperidine ring, a thiopyran ring, a pyridazine ring, a pyrimidine ring, a pyrazine ring, a piperazine ring, a morpholine ring, a triazine ring, a benzofuran ring, an isobenzofuran ring, a benzothiophene ring, an indole ring, an isoindole ring, an indolizine ring, an indoline ring, an isoindoline ring, a chromene ring, a chroman ring, an isochroman ring, a benzopyran ring, a quinoline ring, an isoquinoline ring, a quinolizine ring, a benzoimidazole ring, a benzothiazole ring, an indazole ring, a naphthyridine ring, a quinoxaline ring, a quinazoline ring, a quinazolidine ring, a cinnoline ring, a phthalazine ring, a purine ring, a pteridine ring, a carbazole ring, a xanthene ring, a phenanthridine ring, an acridine ring, a β-carboline ring, a pyrimidine ring, a phenanthroline ring, a thianthrene ring, a phenoxathiin ring, a phenoxazine ring, a phenothiazine ring, and a phenazine ring.

Rings H and I are the same or different and represent a polycyclic aromatic ring. Examples of the polycyclic aromatic ring include the same polycyclic aromatic ring as those represented by the above rings J and K.

In formula (B), $R_{50}$ and $R_{51}$ represent a hydrogen atom or a substituent. When $R_{50}$ and $R_{51}$ are substituents, a group having 1 to 30 carbon atoms is preferred. Examples of the substituent include alkyl groups such as a methyl group, an ethyl group, a butyl group, a hexyl group, an octyl group and a dodecyl group; alkoxy groups such as a methoxy group, an ethoxy group, a butoxy group, a hexyloxy group, an octyloxy group and a dodecyloxy group; aryl groups such as a phenyl and a naphthyl.

Specific examples of the cyclic structure formed by combining $R_{50}$ and $R_{51}$ include structures represented by formulas (a) to (c) shown below.

R30
R31
Y30
Y31
(a)
X30
Y32
Y33
(b)
X31
Y34
Y35
(c)

In formulas (a) to (c), $R_{30}$ and $R_{31}$ are the same or different and represent a hydrogen atom or a substituent. When $R_{30}$ and $R_{31}$ are substituents, the preferable substituent is a group having 1 to 30 carbon atoms.

Examples of the substituent include alkyl groups such as a methyl group, an ethyl group, a butyl group, a hexyl group, an octyl group and a dodecyl group; alkoxy groups such as a methoxy group, an ethoxy group, a butoxy group, a hexyloxy group, an octyloxy group and a dodecyloxy group; and aryl groups such as a phenyl group and a naphthyl group.

$X_{30}$ represents a sulfur atom or a selenium atom. $X_{30}$ is preferably a sulfur atom. $Y_{30}$ to $Y_{35}$ are the same or different and represent a nitrogen atom or =CH-. $Y_{30}$ to $Y_{35}$ are preferably nitrogen atoms. $X_{31}$ represents a sulfur atoms, an oxygen atom, a selenium atom, -N($R_{10}$)- or -$CR_{11}$= $CR_{12}$-. $X_{31}$ is preferably a sulfur atom. $R_{10}$, $R_{11}$ and $R_{12}$ represent a hydrogen atom or a substituent. When $R_{10}$, $R_{11}$ and $R_{12}$ are substituents, examples of the substituent include alkyl groups such as a methyl group, an ethyl group, a butyl group, a hexyl group, an octyl group and a dodecyl group; and aryl groups such as a phenyl group and a naphthyl group.

Ring I may have a substituent other than $R_{50}$ and $R_{51}$ and examples of the substituent include alkyl groups such as a methyl group, an ethyl group, a butyl group, a hexyl group, an octyl group and a dodecyl group; alkoxy groups such as a methoxy group, an ethoxy group, a butoxy group, a hexyloxy group, an octyloxy group and a dodecyloxy group; and aryl groups such as a phenyl group and a naphthyl group. Rings H, J and K may have a substituent, and examples of the substituent include the same substituents as those other than $R_{50}$ and $R_{51}$ as those which may be contained in ring I.

$R_{60}$'s are the same or different and represent a hydrogen atom or a substituent. When $R_{60}$ is a substituent, examples of the substituent include alkyl groups such as a methyl group, an ethyl group, a butyl group, a hexyl group, an octyl group and a dodecyl group; and aryl groups such as a phenyl group and a naphthyl group.

The preferable divalent aromatic group represented by formulas (A) to (C) is a group represented by any one of formulas (2) to (4).

(2) (3) (4)

In formulas (2) to (4), $X_1$ to $X_4$ are the same or different and represent a sulfur atom, an oxygen atom, a selenium atom, $-N(R_{10})-$ or $-CR_{11}=CR_{12}-$. $R_{10}$, $R_{11}$ and $R_{12}$ have the same meanings as defined above, and $X_1$ to $X_4$ are preferably sulfur atoms. $Y_1$ to $Y_8$ are the same or different and represent a nitrogen atom or =CH-. $Y_1$ to $Y_4$ are preferably nitrogen atoms.

$R_1$ to $R_6$ are the same or different and represent a hydrogen atom or a substituent. When $R_1$ to $R_6$ are substituents, the preferable substituent is a group having 1 to 30 carbon atoms. Examples of the substituent include alkyl groups such as a methyl group, an ethyl group, a butyl group, a hexyl group, an octyl group and a dodecyl group; alkoxy groups such as a methoxy group, an ethoxy group, a butoxy group, a hexyloxy group, an octyloxy group and a dodecyloxy group; and aryl groups such as a phenyl and a naphthyl $R_1$ and $R_2$ may combine to form a cyclic structure. $R_3$ and $R_4$ may combine to form a cyclic structure.

Specific examples of the cyclic structure formed by combining $R_1$ and $R_2$ or combining $R_3$ and $R_4$ include a cyclic structure represented by formula (d) and a cyclic structure represented by formula (e).

(d) (e)

In formula (d) and (e), $R_{32}$ and $R_{33}$ are the same or different and represent a hydrogen atom or a substituent. When $R_{32}$ and $R_{33}$ are substituents, examples of the substituent include the same substituents as those represented by the above $R_1$ to $R_6$. $X_{32}$ represents a sulfur atom or a selenium atom. $X_{32}$ is preferably a sulfur atom. $Y_{36}$ to $Y_{39}$ are the same or different and represent a nitrogen atom or =CH-. $Y_{36}$ to $Y_{39}$ are preferably nitrogen atoms.

The groups represented by formulas (2) to (4) are preferably groups represented by formulas (500) to (513):

(500) (501) (502) (503)

(504) (505) (506) (507) (508)

(509) (510) (511) (512) (513)

wherein R has the same meaning as defined in formulas 8 to 131.

The compound of the present invention may have a structure represented by formula (1), and two or more kinds of divalent aromatic groups different from that of formula (1).

It is preferred to have a structure represented by formula (1), a repeating unit represented by formula (300) and groups represented by formulas (A) to (C), and more preferably a repeating unit represented by formula (300) and groups represented by formulas (2) to (4):

(300)

wherein ring A and ring B are the same or different and represent aromatic ring , and $Z_1$ represents a sulfur atom, an oxygen atom, a selenium atom, $-N(R_{20})-$, $-Si(R_{21}R_{22})-$ or - $C(R_{23}R_{29})-$, in which

$R_{20}$ to $R_{24}$ are the same or different and represent a hydrogen atom or a substituent, $R_{21}$ and $R_{22}$ may combine to form a cyclic structure, and $R_{23}$ and $R_{24}$ may combine to form a cyclic structure.

When $R_{20}$ to $R_{24}$ are substituents, examples of the substituent include alkyl groups such as a methyl group, an ethyl group, a butyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group and an eicosyl group; and aryl groups such as a phenyl group and a naphthyl group. $R_{20}$ to $R_{24}$ are preferably hydrocarbon groups, and more preferably alkyl groups. When $R_{20}$ to $R_{24}$ are substituents, the preferable substituent has 4 or more carbon atoms, and more preferably 6 or more carbon atoms.

$Z_1$ is preferably $-Si(R_{21}R_{22})-$ or $-C(R_{23}R_{24})-$, and more preferably $-C(R_{23}R_{24})-$.

Examples of rings A and B include aromatic hydrocarbon rings such as a benzene ring and a naphthalene ring; and aromatic heterocycles such as thiophene. Rings A and B are preferably 4- to 7-membered rings, and more preferably benzene rings or naphthalene rings.

Among repeating units represented by formula (300), a repeating unit represented by formula (300-1):

( 300 - 1 )

wherein $R_{23}$ and $R_{24}$ have the same meanings as defined above is preferred.

The compound of the present invention may be a compound having any molecular weight and is preferably a polymer compound. In the present invention, the polymer compound refers to a compound having a weight average molecular weight of 3,000 or more and a polymer compound having a weight average molecular weight of 3,000 to 10,000,000 is preferably used. When the weight average molecular weight is less than 3,000, defects may be likely caused in the formation of a film upon the production of a device. In contrast, when the weight average molecular weight is more than 10,000,000, solubility in a solvent and applicability upon the production of a device may likely deteriorate. The weight average molecular weight is more preferably from 8,000 to 5,000,000, and particularly preferably from 10,000 to 1,000,000.

The weight average molecular weight in the present invention refers to a polystyrene-equivalent weight average molecular-weight calculated by gel permeation chromatography (GPC) using polystyrene standard samples.

At least one structure represented by formula (1) in the present invention may be contained in the compound. When the compound of the present invention is a polymer compound, the polymer compound preferably contains two or more structures on average per one polymer chain, and more preferably three or more structures on average per one polymer chain.

When the compound of the present invention is used as the device, the device preferably has high solubility in solvent in viewpoint of ease of the production of a device. Specifically, the compound of the present invention preferably has solubility so as to enable the preparation of a solution containing 0.01% by weight or more of the compound, more preferably solubility so as to enable the preparation of a solution containing 0.1% by weight or more of the compound, and still more preferably solubility so as to enable the preparation of a solution containing 0.4% by weight or more of the compound.

When the compound of the present invention has two or more structures represented by formula (1) in one molecule and also has a structure other than the structure represented by formula (1), it is preferred that the structure represented by formula (1) does not continue from the viewpoint of improving photoelectric conversion efficiency.

When the compound of the present invention is a polymer compound, the method of producing a polymer compound is not particularly limited and is preferably a method using Suzuki coupling reaction from the viewpoint of ease of the synthesis of a polymer compound.

Examples of the method using Suzuki coupling reaction include a production method comprising a step of reacting one or more compounds represented by formula (100):

$$Q_1\text{-}E_1\text{-}Q_2 \qquad (100)$$

wherein $E_1$ represents a structure unit including the groups represented by formulas (A) to (C), and $Q_1$ and $Q_2$ are the same or different and represent a boric acid residue ($-B(OH)_2$) or a boric acid ester residue, with one or more compounds represented by formula (200):

$$T_1\text{-}E_2\text{-}T_2 \qquad (200)$$

wherein $E_2$ represents a structure unit including the group represented by formula (1), and $T_1$ and $T_2$ are the same or different and represent a halogen atom, an alkyl sulfonate group, an aryl sulfonate group, or an arylalkyl sulfonate group, in the presence of a palladium catalyst and a base.

In this case, the total of the number of mols of one or more compounds represented by formula (200) used in the reaction is preferably excessive based on the total of the number of mols of one or more compounds represented by formula (100). Assuming that the total of the number of mols of one or more compounds represented by formula (200) used in the reaction is 1 mol, the total of the number of mols of one or more compounds represented by formula

(100) is preferably from 0.6 to 0.99 mol, and more preferably from 0.7 to 0.95 mol.

Examples of the boric acid ester residue include groups represented by formulas shown below:

wherein Me represents a methyl group and Et represents an ethyl group.

In formula (200), examples of the halogen atom in $T^1$ and $T^2$ include a fluorine atom, a chlorine atom, a bromine atom and an iodine atom. From the viewpoint of ease of the synthesis of a polymer compound, the pareferable halogen atom is a bromine atom or an iodine atom, and more preferably a bromine atom.

In formula (200), examples of the alkylsulfonate group in $T^1$ and $T^2$ include a methanesulfonate group, an ethanesulfonate group, and a trifluoromethanesulfonate group. Examples of the arylsulfonate group include a benzenesulfonate group, and a p-toluenesulfonate group. Examples of the arylsulfonate group include a benzylsulfonate group.

Using palladium [tetrakis(triphenylphosphine)], palladium acetates, dichlorobis(triphenylphosphine) palladium (II) and the like as the catalyst, inorganic bases such as potassium carbonate, sodium carbonate and barium hydroxide, organic bases such as triethylamine, and inorganic salts such as cesium fluoride are added in the amount of equivalent or more, and preferably from 1 to 20 equivalents, relative to a monomer as a raw material, and then reacted. Examples of the solvent include N,N-dimethylformamide, toluene, and dimethoxyethane, tetrahydrofuran. The base is added in the form of an aqueous solution, and the reaction may be performed in a two-phase system. When the reaction may be performed in the two-phase system, if necessary, a phase transfer catalyst such as a quaternary ammonium salt may be added. The reaction temperature varies depending on the solvent, and the temperature of around 50 to 160°C is suitably used. Refluxing may be performed by raising the temperature to around a boiling point of the solvent. The reaction time is from around 0.1 to 200 hours. The reaction is performed under an inert atmosphere such as an argon gas or a nitrogen gas in the conditions where the catalyst is not deactivated.

Examples of the palladium catalyst used in the method of producing a polymer compound include palladium [tetrakis(triphenylphosphine)], palladium acetates, and dichiorobis(triphenylphosphire)palladium(II), including a Pd(0) catalyst and a Pd(II) catalyst, for example. From the viewpoint of ease of the reaction (polymerization) operation and the reaction (polymerization) rate, dichlorobis (triphenylphosphine)palladium (II) and palladium acetates are preferred.

The additive amount of the palladium catalyst is not particularly limited and may be an effective amount as the catalyst, and is usually from 0.0001 mol to 0.5 mol, and preferably from 0.0003 mol to 0.1 mol, based on 1 mol of the compound represented by formula (100).

Examples of the base used in the production of a polymer compound include an inorganic base, an organic base, and an inorganic salt.

Examples of the inorganic base include potassium carbonate, sodium carbonate, and barium hydroxide. Examples of the organic base include triethylamine, and tributylamin. Examples of the inorganic salt include cesium fluoride.

The additive amount of the base usually from 0.5 mol to 100 mol, preferably from 0.9 mol to 20 mol, and more preferably from 1 mol to 10 mol, based on 1 mol of the compound represented by formula (100).

When palladium acetates are used as the palladium catalyst, for example, phosphorus compounds such as triphenylphosphine, tri(o-tolyl)phosphine, and tri(o-methoxyphenyl)phosphine can be added as a ligand. In this case, the additive amount of the ligand is usually from 0.5 mol to 100 mol, preferably from 0.9 mol to 20 mol, and more preferably from 1 mol to 10 mol, based on 1 mol of the palladium catalyst.

In the method of producing a polymer compound, the reaction is usually performed in a solvent. Examples of the solvent include N,N-dimethylformamide, toluene, dimethoxyethane, tetrahydrofuran and the like. From the viewpoint of solubility of the polymer compound used in the present invention, toluene and tetrahydrofuran are preferred. The base is added in the form of an aqueous solution, and the reaction may be performed in a two-phase system. When the inorganic salt is used as the base, the base is usually added in the form of an aqueous solution from the viewpoint of solubility of the inorganic salt.

When the base is added in the form of an aqueous solution and the reaction is performed in the two-phase system, if necessary, a phase transfer catalyst such as a quaternary ammonium salt may be added.

The temperature at which the reaction is performed varies according to the solvent and is usually from around 50 to 160°C, and preferably from 60 to 120°C from the viewpoint of an increase in molecular weight of the polymer compound. Refluxing may be performed by raising the temperature to around a boiling point of the solvent.

When the polymerization degree reaches the objective polymerization degree, the reaction may be regarded as the end point thereof, and the time during which the reaction is performed (reaction time) is usually from around 0.1

hours to 200 hours. It is preferably from around 1 hour to 30 hours from the viewpoint of efficiency.

The reaction is performed under an inert atmosphere such as an argon gas or a nitrogen gas in a reaction system in which a Pd(0) catalyst is not deactivated. For example, the reaction is performed in a system which is sufficiently deaerated by an argon gas, a nitrogen gas and the like. Specifically, after deaeration by sufficiently replacing the atmosphere inside a polymerization vessel (a reaction system) with a nitrogen gas, a compound represented by formula (100), a compound represented by formula (200) and dichlorobis (triphenylphosphine) palladium(II) are charged in the polymerization vessel. Furthermore, after deaeration by sufficiently replacing the atmosphere inside the polymerization vessel with a nitrogen gas, a solvent deaerated in advance by bubbling a nitrogen gas, for example, toluene is added. Then, to this solution, a base deaerated in advance by bubbling a nitrogen gas, for example, an aqueous sodium carbonate solution was added dropwise, and then the temperature is raised by heating and the polymerization is performed at a reflux temperature for 8 hours while maintaining an inert atmosphere.

The polystyrene-equivalent number average molecular weight of the polymer compound is preferably from $1 \times 10^3$ to $1 \times 10^8$, and more preferably from $2 \times 10^3$ to $1 \times 10^7$. When the polystyrene-equivalent number average molecular weight is $1 \times 10^3$ or more, it becomes easy to obtain a tough thin film. In contrast, when the polystyrene-equivalent number average molecular weight is $10^8$ or less, it is easy to form a thin film because of high solubility. The polystyrene-equivalent weight average molecular weight is preferably $3.0 \times 10^3$ or more, and more preferably $1.0 \times 10^4$ or more. The number average molecular weight is preferably from $1.0 \times 10^4$ to $1.0 \times 10^7$, and particularly preferably from $1.0 \times 10^4$ to $1.0 \times 10^6$.

When a polymerization active group remains as it is in a terminal group of a polymer compound which can be used in the present invention, characteristics and lifetime of a device obtained when used in the production of the device may deteriorate, and therefore the terminal group may be protected with a stable group. The terminal group having a conjugated bond contiguous with a conjugated structure of a main chain is preferred and, for example, it may have a structure which combines with an aryl group or a heterocyclic group through a vinylene group. Specifically, it is exemplified by the substituents described in Chemical Formula 10 of JP-A-9-45478 and the like.

The light absorption end wavelength in the present invention means the value determined by the following method.

In the measurement, a spectrophotometer capable of operating in an ultraviolet, visible or near infrared wavelength range (for example, UV-visible near infrared spectrophotometer JASCO-V670 manufactured by JASCO Corporation) is used. When JASCO-V670 is used, since a measurable wavelength is in a range from 200 to 1,500 nm, the measurement is made in the above wavelength range. First, an absorption spectrum of a substrate to be used in the measurement is measured. As the substrate, a quartz substrate, a glass substrate, and the like are used. Next, a thin film containing the compound of the present invention is formed from a solution containing the compound of the present invention or a melt containing the compound of the present invention is formed on the substrate. In the formation of a film from a solution, drying is performed after the formation of the film. Then, an absorption spectrum of a laminate of the thin film and the substrate is obtained. A difference between the absorption spectrum of the laminate of the thin film and the substrate, and the absorption spectrum of the substrate is obtained as the absorption spectrum of the thin film.

With respect to the absorption spectrum of the thin film, the ordinate denotes an absorbance of the present invention and the abscissas denotes a wavelength. It is desired to adjust the film thickness of the thin film so that the absorbance of the largest absorption peak is around 0.5 to 2. It is assumed that an absorbance of an absorption peak at the longest wavelength among the absorption peaks is 100%, and that an intersection point between a straight line parallel to the abscissas (wavelength axis) including 50% of the absorbance, and the absorption peak, which is on a wavelength longer than the peak wavelength of the absorption peak, is a first point. It is assumed that an intersection point between a straight line parallel to a wavelength axis including 25% of the absorbance, and the absorption peak, which is on a wavelength longer than the peak wavelength of the absorption peak, is a second point. An intersection point between a straight line connecting the first point and the second point, and a base line is defined as a light absorption end wavelength. As used herein, the base line refers to a straight line connecting a third point on an absorption spectrum having a wavelength which is 100 nm longer than a base wavelength and a fourth point on an absorption spectrum having a wavelength which is 150 nm longer than a base wavelength, assuming that the base wavelength is a wavelength of an intersection point between a straight line parallel to a wavelength axis containing 10% of an absorbance of an absorption peak (100%) at the longest wavelength and the absorption peak, a wavelength of the intersection point being longer than the peak wavelength of the absorption peak.

From the viewpoint of an increase in light absorption end wavelength, it is preferred that the compound of the present invention has, in addition to a structure represented by formula (1), a divalent aromatic group. The preferable divalent aromatic group is a divalent aromatic group in which two hydrogen atoms have been removed from the above aromatic fused ring formed by the condensation of two or more aromatic rings selected from the group consisting of an aromatic 7-membered ring, an aromatic 6-membered ring and an aromatic 5-membered ring and, in the divalent aromatic group, an divalent aromatic group in which the number of the atoms constituting the aromatic fused ring existing in the shortest path between two atoms having a bond is 3 or 4, including atoms having a bond (bonding site) is preferred.

When the compound of the present invention has an divalent aromatic group in which the number of the atoms constituting the aromatic fused ring existing in the shortest path between two atoms having a bond is 3 or 4, including atoms having a bond (bonding site), a band gap between HOMO and LUMO of the compound becomes narrow, and thus the light absorption end wavelength becomes a long wavelength.

It is preferred that the compound used suitably in a photoelectric conversion device includes a repeating unit represented by formula (1-1) and also includes a repeating unit represented by any one of formulas (2-1), (3-1) and (4-1):

( 1-1 )

( 2 -1) ( 3 -1) ( 4-1 )

wherein $R_1$ to $R_6$, $X_1$ to $X_4$, $Y_1$ to $Y_4$ have the same meanings as defined above.

In this case, the total of the formula weight of a structure represented by formula (1-1) is usually from 0.0001 to 0.5, preferably from 0.0005 to 0.4, and more preferably from 0.001 to 0.3, based on the molecular weight of the compound of the present invention.

The total of the formula weight of structures represented by formulas (2-1), (3-1) and (4-1) is usually from 0.0001 to 0.5, preferably from 0.0005 to 0.4, and more preferably from 0.001 to 0.3, based on the molecular weight of the present invention.

The compound of the present invention can exhibit high carrier (electron or hole) transportability and is therefore capable of transporting electrons and holes injected from electrodes, or charges generated by light absorption when an organic thin film containing the compound is used in a device. The organic thin film can be applied to various devices such as photoelectric conversion devices and an organic thin film transistor, making use of these characteristics. These devices will be individually described below.

<Device>

The device of the present invention is a device comprising a first electrode and a second electrode, and an active layer between the first electrode and the second electrode, the active layer containing the compound of the present invention.

Examples of the device of the present invention include a device in which at least one layer of the active layer is a layer made of only the compound of the present invention.

Examples of the device of the present invention include a photoelectric conversion device, an organic thin film transistor (device) and an organic electroluminescence device (organic EL device).

The device of the present invention can be produced by a production method comprising a step of applying a solution containing the compound of the present invention and a solvent on a first electrode by an applying method to form an active layer, and a step of forming a second electrode on the active layer.

<Photoelectric Conversion device>

The photoelectric conversion device of the present invention comprises one or more active layers containing the composition of the present invention between a pair of electrodes, at least one of which is transparent or translucent.

In preferred mode of the photoelectric conversion device of the present invention, the photoelectric conversion

device comprises a pair of electrodes, at least one of which is transparent or translucent, and an active layer formed from an organic composition of a p-type organic semiconductor and an n-type organic semiconductor. It is preferred that the compound of the present invention is used as p-type organic semiconductor. A motion mechanism of the photoelectric conversion device of this mode will be described. Energy of light made incident from the transparent or translucent electrode is absorbed by the electron-accepting compound (n-type organic semiconductor) such as a fullerene derivative and/or the electron-donating compound (p-type organic semiconductor) such as a compound of the present invention to form excitons in which electrons and holes combine. When the excitons thus formed move and reach a heterojunction interface where the electron-accepting compound is adjacent to the electron-donating compound, electrons and holes are separated due to a difference in each of HOMO energy and LUMO energy at the interface to generate charges (electrons and holes) capable of independently moving. The charges thus generated can be extracted outside as electric energy (current) by respectively moving to the electrode.

The photoelectric conversion device of the present invention is usually formed on a substrate. This substrate may be any substrate as long as it forms the electrode and does not change when a layer of an organic substance is formed. Examples of the material of the substrate include glass, plastic, polymer film, and silicone. In a case of an opaque substrate, the opposite electrode (i.e., an electrode which is distal from the substrate) is preferably transparent or translucent.

Another aspect of the photoelectric conversion device of the present invention is a photoelectric conversion device which comprises a first active layer containing the compound of the present invention and a second active layer containing an electron-accepting compound such as a fullerene derivative, adjacent to the first active layer, between a pair of electrodes, at least one of which is transparent or translucent.

Examples of the transparent or translucent electrode material include a conductive metal oxide film, and a translucent metal thin film. Specifically, a film (NESA, etc.) formed of conductivity materials composed of indium oxide, zinc oxide, tin oxide, and a complex thereof such as indium tin oxide (ITO) or indium zinc oxide, gold, platinum, silver, copper, and the like are used, and ITO, indium zinc oxide and tin oxide are preferred. Examples of the method of producing an electrode include a vacuum deposition method, a sputtering method, an ion plating method, and a plating method. As the electrode material, an organic transparent conductive film formed of polyaniline and a derivative thereof, polythiophene and a derivative thereof, and the like may be used. Furthermore, metal, a conductive polymer, and the like can be used as the electrode material. Preferably, one electrode of a pair of electrodes is formed of a material having a small work function. For example, it is possible to use metals such as lithium, sodium, potassium, rubidium, cesium, magnesium, calcium, strontium, barium, aluminum, scandium, vanadium, zinc, yttrium, indium, cerium, samarium, europium, terbium, ytterbium, and an alloy of two or more metals, or an alloy of one or more of metals and one or more of gold, silver, platinum, copper, manganese, titanium, cobalt, nickel, tungsten and titanium, and graphite or a graphite intercalation compound.

Examples of the alloy include a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy.

As means adapted to improve photoelectric conversion efficiency, an additional intermediate layer other than the active layer may be used. Examples of the material used as an intermediate layer include alkali metals such as lithium fluoride, halides of alkaline earth metal, oxides such as titanium oxide, and PEDOT (poly-3,4-ethylenedioxythiophene).

<Active Layer>

The active layer may contain the compounds of the present invention alone, or may contain two or more kinds of them in combination. In order to enhance hole transportability of the active layer, it is possible to be used by mixing a compound other than the compound of the present invention in the active layer as an electron-donating compound and/or an electron-accepting compound. The electron-donating compound and the electron-accepting compound are relatively determined from an energy level of energy levels of these compounds.

Examples of the electron-donating compound include, in addition to the compound of the present invention, a pyrazoline derivative, an arylamine derivative, a stilbene derivative, a triphenyldiamine derivative, oligothiophene and a derivative thereof, polyvinylcarbazole and a derivative thereof, polysilane and a derivative thereof, a polysiloxane derivative having an aromatic amine in a side chain or a main chain, polyaniline and a derivative thereof, polythiophene and a derivative thereof, polypyrrole and a derivative thereof, polyphenylenevinylene and a derivative thereof, and polythienylenevinylene and a derivative thereof.

Examples of the electron-accepting compound include, in addition to the compound of the present invention, a carbon material, metal oxide such as titanium oxide, an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene

and a derivative thereof, diphenoquinone derivative, 8-hydroxyquinoline and a metal complex of a derivative thereof, polyquinoline and a derivative thereof, polyquinoxaline and a derivative thereof, polyfluorene and a derivative thereof, a phenanthrene derivative such as 2,9-dimethyl-4,7-diphenyl-1,10-phenanthroline (Bathocuproin), fullerene, and a fullerene derivative; and the electron-accepting compound is preferably titanium oxide, carbon nano-tube, fullerene or a fullerene derivative, and particularly preferably fullerene or a fullerene derivative. Examples of fullerene and a fullerene derivative include $C_{60}$, $C_{70}$, $C_{76}$ $C_{78}$, $C_{84}$, and a derivative thereof. Example of the fullerene derivative includes the followings.

OCH3
O
C12H25O
OC12H25
N
O
O
S
OH
N
C11H23
OCH3
O
OCH3
O
H3CO
O

Examples of the fullerene derivative include [6,6]-phenyl C61 butyric acid methyl ester (C60PCBM), [6,6]-phenyl

C71 butyric acid methyl ester (C70PCBM), [6,6]-phenyl C85 butyric acid methyl ester (C84PCBM), and [6,6]-thienyl C61 butyric acid methyl ester.

When the compound of the present invention and the fullerene derivative are contained in the active layer, the amount of the fullerene derivative is preferably from 10 to 1,000 parts by weight, and more preferably from 20 to 500 parts by weight, based on 100 parts by weight of the compound of the present invention.

Usually, the thickness of the active layer is preferably from 1 nm to 100 μm, more preferably from 2 nm to 1,000 nm, still more preferably from 5 nm to 500 nm, and further preferably from 20 nm to 200 nm.

The active layer may be formed by any method and examples thereof include a method of forming a film from a solution containing a polymer compound, and a method of forming a film using a vacuum deposition method.

<Method for Production of Photoelectric Conversion device>

Preferred method for producing a photoelectric conversion device of the present invention is a method for producing a device including a first electrode and a second electrode, and an active layer between the first electrode and the second electrode, the method including the steps of applying a solution containing the compound according to any one of claims 1 to 6 and a solvent on the first electrode by an applying method to form the active layer, and forming a second electrode on the active layer.

The solvent used in the formation of a film from a solution is required to dissolve the compound of the present invention. Examples of this solvent include unsaturated hydrocarbon solvents such as toluene, xylene, mesitylene, tetralin, decalin, bicyclohexyl, n-butylbenzene, sec-butylbenzene and tert-butylbenzene; halogenated saturated hydrocarbon solvents such as carbon tetracloride, chloroform, dichloromethane, dichloroethane, chlorobutane, bromobutane, chloropentane, bromopentane, chlorohexane, bromohexane, chlorocyclohexane and bromocyclohexane; halogenated unsaturated hydrocarbon solvents such as chlorobenzene, dichlorobenzene and trichlorobenzene; ether solvents such as tetrahydrofuran and tetrahydropyran. The compound of the present invention can be usually dissolved in the solvent in the concentration of 0.005% by weight or more, and preferably 0.1% by weight or more.

When a film is formed using the solution, it is possible to use applying methods such as a slit coating method, a knife coating method, a spin coating method, a casting method, a microgravure coating method, a gravure coating method, a bar coating method, a roll coating method, a wire bar coating method, a dip coating method, a spray coating method, a screen printing method, a gravure printing method, a flexo printing method, an offset printing method, an inkjet coating method, a dispenser printing method, a nozzle coating method and a capillary coating method, and a slit coating method, a capillary coating method, a gravure coating method, a microgravure coating method, a bar coating method, a knife coating method, a nozzle coating method, an inkjet coating method or a spin coating method is preferred.

From the viewpoint of film forming properties, a surface tension at 25°C of the solvent is preferably more than 15 mN/m, more preferably more than 15 mN/m and less than 100 mN/m, and still more preferably more than 25 mN/m and less than 60 mN/m.

<Organic Thin Film Transistor>

The compound of the present invention can also be used in an organic thin film transistor. The organic thin film transistor includes, for example, an organic thin film transistor with a constitution including a source electrode and a drain electrode, an organic semiconductor layer (active layer) serving as a current path formed between these electrodes, and a gate electrode for controlling the amount of current which passes through this current path, and the organic semiconductor layer is formed from the above organic thin film. Examples of such an organic thin film transistor include a field effect type organic thin film transistor, and a static induction type organic thin film transistor.

It is preferred that field effect type organic thin film transistor is provided with a source electrode and a drain electrode, an organic semiconductor layer (active layer) serving as a current path formed between these electrodes, a gate electrode for controlling the amount of current which passes through this current path, and an insulating layer arranged between the organic semiconductor layer and the gate electrode.

It is particularly preferred that the source electrode and drain electrode are arranged in contact with the organic semiconductor layer (active layer) and also the gate electrode is arranged to sandwich the insulating layer in contact with the organic semiconductor layer. In the field effect type organic thin film transistor, the organic semiconductor layer is formed of an organic thin film made from the compound of the present invention.

It is preferred that the static induction type organic thin film transistor comprises a source electrode and a drain electrode, an organic semiconductor layer (active layer) serving as a current path formed between these electrodes, a gate electrode for controlling the amount of a current which passes through this current path, and this gate electrode is provided in the organic semiconductor layer. It is particularly preferred that the source electrode, the drain electrode, and the gate electrode provided in the organic semiconductor layer are provided in contact with the organic semiconductor layer. Herein, the structure of the gate electrode may be a structure in which a path of a current flowing from the source

electrode to the drain electrode is formed and also the amount of a current flowing through the current path can be controlled by a voltage applied to the gate electrode. For example, a comb-shaped electrode is exemplified. Also in the static induction type organic thin film transistor, the organic semiconductor layer is formed of an organic thin film made from the compound of the present invention.

<Applications of Device>

The photoelectric conversion device of the present invention can be operated as an organic thin film solar battery when it is irradiated with light such as sunlight from transparent or translucent electrodes to generate a photoelectromotive force between the electrodes. It is also possible to use as an organic thin film solar battery module by integrating a plurality of organic thin film solar batteries.

It is also possible to operate as an organic optical sensor when a photocurrent flows by irradiation with light from transparent or translucent electrodes in a state where a voltage is applied or not between the electrodes. It is also possible to use as an organic image sensor by integrating a plurality of organic optical sensors.

The above organic thin film transistor can be used, for example, as a pixel driving device or the like which is used to control uniformity of screen luminance and a screen rewriting speed of an electrophoretic display, a liquid crystal display, an organic electroluminescence display and the like.

<Solar Battery Module>

An organic thin film solar battery can have a module structure which is basically the same as that of a conventional solar battery module. The solar battery module commonly has a structure in which a cell is formed on a supporting substrate of metal, ceramic or the like and a filling resin, a protective glass or the like is coated thereon, and light is captured from the opposite side of the supporting substrate. It is also possible to have a structure in which a transparent material such as reinforced glass is used as the material of a supporting substrate and a cell is formed thereon, and light is captured from the side of a transparent supporting substrate. Specifically, a module structure referred to as a super straight type, a substrate type or a potting type, substrate integrated type module structure used in an amorphous silicone solar battery or the like, and the like are known. In the organic thin film solar battery of the present invention, these module structures can be appropriately selected according to purposes of use, places of use and environment.

Typical super straight type or substrate type module has a structure in which cells are arranged at given intervals between supporting substrates, one side or both sides is/are transparent, subjected to an anti-reflection treatment, and adjacent cells are connected to each other by a metal lead or flexible wiring, and also a current collecting electrode is arranged at an external peripheral portion and electric power generated is extracted outside. Between the substrate and cells, various plastic materials such as ethylene vinyl acetate (EVA) may be used in the form of a film or a filling resin for the purposes for the purpose of protecting cells and improving current collecting efficiency. When used in the place where coating of the surface with a hard material is not required, for example, a place where exposed to little impact from the outside, a surface protective layer is formed of a transparent plastic film or a protective function is imparted by curing the filling resin, and thus a supporting substrate of one side can be omitted.

The periphery of the supporting substrate is fixed with a frame made of metal in a sandwich shape so as to ensure seal inside and rigidity of a module, and the space between the supporting substrate and the frame is sealed with a sealing material. A solar battery can also be formed on a curved surface when a flexible material is used as the cell per se, or a supporting substrate, a filling material and a sealing material.

In a case of a solar battery using a flexible substrate such as a polymer film, a battery body can be produced by sequentially forming cells while feeding a roll-shaped substrate, cutting into a desired size and then sealing a peripheral portion with a flexible and moisture-resistant material. It is also possible to employ a module structure called "SCAF" described in Solar Energy Materials and Solar Cells, 48, p383-391. Furthermore, a solar battery using a flexible substrate can also be used in a state of being bonded and fixed to a curved glass or the like.

<Organic Electroluminescence Device>

The compound of the present invention can also be used in an organic electroluminescence device (an organic EL device). It is preferred that the organic EL device includes an emissive layer between a pair of electrodes, at least one of which is transparent or translucent, and the composition of the present invention is contained in the emissive layer. In the emissive layer, in addition to the compound of the present invention, a charge transporting material (which means a generic term of an electron transporting material and a hole transporting material) may be contained. Examples of the organic EL device include a device comprising an anode, an emissive layer and a cathode; a device comprising an anode, an emissive layer, an electron transporting layer and a cathode, the electron transporting layer containing an electron transporting material being formed between the cathode and the emissive layer adjacent to the emissive layer;

a device comprising an anode, a positive hole transporting layer, an emissive layer and a cathode, the positive hole transporting layer containing a positive hole transporting material being formed between the anode and the emissive layer adjacent to the emissive layer; a device comprising an anode, a positive hole transporting layer, an emissive layer, an electron transporting layer and a cathode; and the like. The compound of the present invention can also be used in a positive hole transporting layer and an electron transporting layer.

EXAMPLES

Examples will be illustrated so as to describe the present invention in more detail, but the present invention is not limited thereto.

(Reference Example 1) Production of 2-bromo-3-dodecylthiophene

Under an argon atmosphere, 15.0 g (59.4 mol) of 3-dodecylthiophene (manufactured by Aldrich Corporation) and 50 ml of dry N,N-dimethylformamide (hereinafter sometimes referred to as DMF) were charged in a 200 ml three-necked flask to obtain a uniform solution. While maintaining the flask at 0°C, a DMF (100 ml) solution of 10.6 g (59.3 mmol) of N-bromosuccinimide (hereinafter sometimes referred to as NBS) was added dropwise over 1 hour. After dropwise addition, while maintaining the flask at 0°C, stirring was performed for 6 hours. Thereafter, the reaction solution was poured into 400 ml of water, and the solution was extracted five times with 50 ml of diethyl ether. The oil layer was washed three times with water and dried over magnesium sulfate, and then solvent was distilled off under reduced pressure to obtain 17.8 g of 2-bromo-3-dodecylthiophene as the objective product. 1H NMR: δ 7.18 (d, 1H), 6.79 (d, 1H), 2.57 (t, 2H), 1.57-1.29 (m, 20H), 0.88 (t, 3H).

(Reference Example 2) Production of 3-dodecylthiophene-2-carbaldehyde

Under an argon atmosphere, 2.40 g (98.7 mmol) of metallic magnesium was charged in a 500 ml three-necked flask and magnesium was activated for one hour by heating at 150°C while stirring using a stirrer chip. After cooling to room temperature, dry tetrahydrofuran (THF) (96 ml) was charged and 50 mg of iodine was added, and also a solution prepared by dissolving 24.0 g (72.4 mmol) of 2-bromo-3-dodecylthiophene synthesized in Reference Example 1 in 96 ml of THF was added dropwise at room temperature over 30 minutes. After the dropwise addition, the mixture was refluxed for 30 minutes and then cooled to room temperature, and 10.5 g (144 mmol) of DMF was added dropwise over 15 minutes. After stirring at room temperature for 2 hours, hydrochloric acid was added to the reaction system until the pH becomes about 2, followed by extraction five times with ethyl acetate. The oil layer was washed with water, saturated brine and water and dried over magnesium sulfate, and then the solvent was distilled off by an evaporator to obtain 20.4 g of a crude product. This crude product was purified by silica gel column (hexane/ethyl acetate = 10/1 (volume ratio)) to obtain 18.2 g (64.9 mmol, yield: 89.6%) of 3-dodecylthiophene-2-carbaldehyde as the objective product. 1H NMR: δ = 10.0(s, 1H), 7.64(d, 1H), 7.01(d, 1H), 2.96(t, 2H), 1.69-1.26(m, 20H), 0.88(t, 3H).

(Reference Example 3) Production of 2,5-bis(3-dodecylthiophen-2-yl)thiazolo[5,4-d]thiazole

In a 100 ml flask, 18.0 g (64.0 mmol) of 3-dodecylthiophene-2-carboaldehyde synthesized in Reference Example 2 and 2.56 g of rubeanic acid (manufactured by Tokyo Chemical Industry Co., Ltd.) were charged and then heated at 200°C for 6 hours. After the reaction, the reaction solution was cooled to room temperature, diluted with a solvent of hexane/ethyl acetate = 10/1 (volume ratio) and then filtered with celite. The filtrate was concentrated and purified by a silica gel column (toluene/hexane = 5/5 (vol/vol) to obtain 5.2 g (8.08 mmol) of 2,5-bis(3-dodecylthlophen-2-yl)thiazolo [5,4-d]thiazole as the objective product. 1H NMR: δ = 7.35(d, 2H), 6.99(d, 2H), 2.97(t, 4H), 1.72-1.26(m, 40H), 0.88(t, 6H).

(Reference Example 4) Production of 2,5-bis(5-bromo-3-dodecylthiophen-2-yl)thiazolo[5,4-d]thiazole

Under an argon atmosphere, 3.28 g (5.10 mol) of 2,5-bis(3-dodecylthiophen-2-yl)thiazolo[5,4-d]thiazole synthesized above and 140 ml of dry DMF were charged in a 100 ml three-necked flask and then dissolved with stirring at 50°C. To this solution, a dry DMF (50 ml) solution of 1.91 g (5.10 mmol) of NBS was added dropwise at 50°C over 30 minutes, followed by stirring at 50°C for 4 hours.

After the reaction, the solution was cooled to room temperature, extracted five times with chloroform and dried over magnesium sulfate, and then the solvent was distilled off by an evaporator to obtain a crude product. This crude product was purified by a silica gel column (hexane/toluene = 7/3 (volume ratio)) to obtain 3.80 g (4.74 mmol) of 2,5-bis (5-bromo-3-dodecylthiophen-2-yl)thiazolo[5,4-d]thiazole as the objective product. 1H NMR: δ = 6.93(s, 2H), 2.86(t, 4H), 1.68-1.26(m, 40H), 0.88(t, 6H).

Example 1 (Production of Polymer A)

Using 2,5-bis(5-bromo-3-dodecylthiophen-2-yl)thiazolo[5,4-d]thiazole (monomer A) produced in aforementioned Reference Example 4, 2,1,3-benzothiadiazole-4,7-bis(boronic acid pinacol ester) (monomer B) (manufactured by Aldrich Corporation) and 9,9-dioctylflucrene-2,7-diboronic acid (monomer C) (manufactured by American Dye Source, Inc.,) a polymer A was produced in the following manner.

Monomer A Monomer B Monomer C

Under an argon atmosphere, 200 mg (0.250 mmol) of a monomer A, 48.5 mg (0.125 mmol) of a monomer B, 66.2 mg (0.125 mmol) of a monomer C, 108 mg of methyltrialkylammonium chloride (manufactured by Aldrich Corporation under the trade name of Aliquat336 (registered trademark)) and 11 mL of toluene were charged in a reaction vessel and the atmosphere inside the reaction vessel was sufficiently deaerated by bubbling using argon. Furthermore, 1.20 mg (0.00534 mol) of palladium acetate, 6.10 mg (0.0173 mmol) of tris(methoxyphenyl)phosphine and 2.4 mL of the deaerated aqueous sodium carbonate solution (16.7% by weight) were added and the mixture was refluxed for 5 hours. Next, 60.0 mg of phenylboric acid was added to the obtained reaction solution, followed by refluxing for 2 hours. Furthermore, 10 ml of an aqueous sodium diethyldithiocarbamate solution (9.1% by weight) was added, followed by refluxing for 2 hours.

After the completion of the reaction, the reaction solution was cooled to around room temperature (25°C) and then the obtained reaction solution was left to stand and the separated toluene layer was recovered. The toluene layer was washed twice with 10 mL of water, washed twice with 3% acetic acid aqueous solution, and then washed twice with 10 mL of water. The obtained toluene layer was poured into methanol and then the precipitate thus obtained was recovered. This precipitate was dried under reduced pressure and then dissolved in chloroform. Next, the obtained chloroform solution was filtered and insolubles were removed, and then the solution was purified by passing through an alumina column. The obtained chloroform solution was concentrated under reduced pressure and poured into methanol, and then the precipitate thus obtained was recovered. This precipitate was washed with methanol and then dried under reduced pressure to obtain 101 mg of a polymer. Hereinafter, this polymer is referred to as a polymer A. The polymer A had a polystyrene-equivalent weight average molecular weight of 20,700 and a polystyrene-equivalent number average molecular weight of 7,000. The polymer A showed a light absorption end wavelength of 738 nm.

Example 2 (Production of Polymer B)

The same operation was performed, except that 200 mg (0.250 mmol) of the monomer A, 63 mg (0.162 mmol) of the monomer B and 46 mg (0.0873 mmol) of the monomer C were used in Example 1, a polymer B was produced. The polymer B had a polystyrene-equivalent weight average molecular weight of 11,000 and a polystyrene-equivalent number average molecular weight of 4,300. The polymer B showed a light absorption end wavelength of 734 nm.

Example 3 (Production of Polymer C)

The same operation was performed, except that 200 mg (0.250 mmol) of the monomer A and 48.5 mg (0.125 mmol) of the monomer B were used and 73.9 mg (0.125 mmol) of 9,9-didodecylfluorene-2,7-diboronic acid (monomer D) (manufactured by Aldrich Corporation):

Monomer D

was used in place of the monomer C in Example 1, to produce a polymer C. The polymer C had a polystyrene-equivalent weight average molecular weight of 11,000 and a polystyrene-equivalent number average molecular weight of 4,900. The polymer C showed a light absorption end wavelength of 738 nm.

Example 4 (Production and Evaluation of Composition and Organic ThinFilm Solar Battery)

A glass substrate with a 150 nm thick ITO film formed by a sputter method was subjected to a surface treatment by an ozone UV treatment. Next, a polymer A and a fullerene C60PCBM (phenyl C61-butyric acid methyl ester, manufactured by Frontier Carbon Corporation) (a weight ratio of polymer A/C60PCBM = 1/3) were dissolved in orthodichlorobenzene (the total of the weight of the polymer A and the weight of C60PCBM was 2.0% by weight) to produce a composition ink 1. The composition ink 1 was applied on the substrate by spin coating to form an organic film containing the polymer A (film thickness: about 100 nm). A rotary speed of a spin coater is 1,500 rpm. The organic film thus formed showed a light absorption end wavelength of 745 nm. Thereafter, lithium fluoride was deposited on the organic film in a thickness of 2 nm by a vacuum deposition machine, and then A1 was deposited thereon in a thickness of 100 nm. The obtained organic thin film solar battery had a shape of square measuring 2 mm $\times$ 2 mm. The obtained organic thin film solar battery was irradiated with light having a given irradiance using a solar simulator (manufactured by BUNKOUKEIKI Co., Ltd. under the trade name of OTENTO-SUNII:AM1.5G filter, irradiance: 100 mW/cm$^2$). The current and voltage generated were measured and a photoelectric conversion efficiency, a short-circuit current density, an open circuit voltage and a fill factor were determined. Jsc (short-circuit current density) was 5.12 mA/cm$^2$, Voc (open circuit voltage) was 0.85 V, ff (fill factor) was 0.57, and photoelectric conversion efficiency ($\eta$) was 2.48%. Example 5 (Production and Evaluation of Composition and Organic Thin-Film Solar Battery)

In the same manner as in Example 4, except that a weight ratio of mixing a polymer A with C60PCBM was set at 1/1, an organic thin film solar battery were produced, and then a photoelectric conversion efficiency, a short-circuit current density, an open circuit voltage and a fill factor were determined. The results are shown in Table 1. Example 6 (Production and Evaluation of Composition and Organic Thin-Film Solar Battery)

In the same manner as in Example 4, except that a weight ratio of mixing a polymer A with C60PCBM was set at 1/2, an organic thin film solar battery were produced, and then a photoelectric conversion efficiency, a short-circuit current density, an open circuit voltage and a fill factor of the organic thin film solar battery were determined. The results are shown in Table 1.

Example 7 (Production and Evaluation of Composition and Organic Thin-Film Solar Battery)

In the same manner as in Example 4, except that a weight ratio of mixing a polymer A with C60PCBM was set at 1/4, an organic thin film solar battery were produced, and then a photoelectric conversion efficiency, a short-circuit current density, an open circuit voltage and a fill factor of the organic thin film solar battery were determined. The results are shown in Table 1.

Example 8 (Production and Evaluation of Composition and Organic Thin-Film Solar Battery)

In the same manner as in Example 4, except that a weight ratio of mixing a polymer A with C60PCBM was set at 1/5, an organic thin film solar battery were produced, and then a photoelectric conversion efficiency, a short-circuit current density, an open circuit voltage and a fill factor of the organic thin film solar battery were determined. The results are shown in Table 1.

Table 1: Photoelectric Conversion Device Evaluation Results

| | Example 5 | Example 6 | Example 7 | Example 8 |
|---|---|---|---|---|
| Short-circuit current density (mA/cm$^2$) | 4.84 | 5.51 | 4.70 | 4.30 |
| Open circuit voltage (V) | 0.89 | 0.87 | 0.85 | 0.83 |
| Fill factor | 0.53 | 0.61 | 0.61 | 0.63 |
| Conversion efficiency (%) | 2.31 | 2.93 | 2.44 | 2.25 |

Example 9 (Production and Evaluation of Composition and Organic Thin-Film Solar Battery)

In the same manner as in Example 5, except that a polymer B was used in place of a polymer A, an organic thin film solar battery were produced, and then a photoelectric conversion efficiency, a short-circuit current density, an open circuit voltage and a fill factor were determined. The results are shown in Table 2.

Example 10 (Production and Evaluation of Composition and Organic Thin-Film Solar Battery)

In the same manner as in Example 9, except that a weight ratio of mixing a polymer B with C60PCBM was set at 1/3, an organic thin film solar battery were produced, and then a photoelectric conversion efficiency, a short-circuit current density, an open circuit voltage and a fill factor were determined. The results are shown in Table 2.

Example 11 (Production and Evaluation of Composition and Organic Thin-Film Solar Battery)

In the same manner as in Example 9, except that a weight ratio of mixing a polymer B with C60PCBM was set at 1/5, an organic thin film solar battery were produced, and then a photoelectric conversion efficiency, a short-circuit current density, an open circuit voltage and a fill factor were determined. The results are shown in Table 2.

Table 2:

| | Example 9 | Example 10 | Example 11 |
|---|---|---|---|
| Short-circuit current density (mA/cm$^2$) | 4.49 | 4.56 | 4.31 |
| Open circuit voltage (V) | 0.86 | 0.84 | 0.81 |
| Fill factor | 0.58 | 0.59 | 0.62 |
| Conversion efficiency (%) | 2.23 | 2.24 | 2.17 |

Example 12 (Production and Evaluation of Composition and Organic Thin-Film Solar Battery)

In the same manner as in Example 5, except that a polymer C was used in place of a polymer A, an organic thin film solar battery were produced, and then a photoelectric conversion efficiency, a short-circuit current density, an open circuit voltage and a fill factor were determined. The results are shown in Table 3.

Table 3:

| | Example 12 |
|---|---|
| Short-circuit current density (mA/cm$^2$) | 2.63 |
| Open circuit voltage (V) | 0.97 |
| Fill factor | 0.51 |
| Conversion efficiency (%) | 1.29 |

Example 13 (Production and Evaluation of Composition and Organic Thin-Film Solar Battery)

In the same manner as in Example 6, except that a C70PCBM (phenyl C71-butyric acid methyl ester, manufactured by Frontier Carbon Corporation) was used in place of a C60PCBM, an organic thin film solar battery were produced, and then a photoelectric conversion efficiency, a short-circuit current density, an open circuit voltage and a

fill factor were determined. The results are shown in Table 4.

Table 4:

| | Example 13 |
|---|---|
| Short-circuit current density ($mA/cm^2$) | 7.29 |
| Open circuit voltage (V) | 0.85 |
| Fill factor | 0.58 |
| Conversion efficiency (%) | 3.57 |

Reference Example 5 (Production of Polymer E)

(E)

(F)

A compound (E) (0.949 g), 1.253 g of a compound (F), 0.30 g of methyltrialkylammonium chloride (manufactured by Aldrich Corporation under the trade name of Aliquat336(registered trademark)) and 3.4 mg of dichlorobis(triphenylphosphine) palladium(II) were charged in a reaction vessel and the atmosphere inside the reaction vessel was replaced by an argon gas. Toluene (45ml) deaerated in advance by bubbling an argon gas was added to this reaction vessel. Next, 10 ml of an aqueous 16.7% by weight sodium carbonate solution deaerated in advance by bubbling the argon gas was added dropwise to this solution under stirring, followed by refluxing for 12 hours. Next, the reaction solution was cooled and a mixed phenylboric acid (0.1 g)/tetrahydrofuran (0.5 ml) solution was added, followed by refluxing for 2 hours. The reaction was performed under an argon gas atmosphere. After completion of the reaction, the reaction solution was cooled and 60 g of toluene was added to this reaction solution. This reaction solution was left to stand and subjected to liquid separation, and then a toluene solution was recovered. Next, this toluene solution was filtered to remove insolubles. Next, this toluene solution was purified by passing through an alumina column. Next, this toluene solution was poured into methanol and purified by reprecipitation, and the precipitate thus obtained was recovered. Next, this precipitate was dried under reduced pressure and then dissolved again in toluene. Next, this toluene solution was filtered and then this toluene solution was purified by passing through an alumina column. Next, this toluene solution was poured into methanol and purified by reprecipitation, and the precipitate thus obtained was recovered. This precipitate was washed with methanol and then dried under reduced pressure to obtain 0.5 g of a polymer E. The polymer E had a polystyrene-equivalent weight average molecular weight of $9.9 \times 10^4$ and a polystyrene-equivalent number average molecular weight of $6.1 \times 10^4$. A repeating unit contained in the polymer E, estimated from charging, is as follows.

Comparative Example 1 (Evaluation of Photoelectric Conversion Device of Polymer E)

The polymer E was dissolved in xylene in the concentration of 0.75% (% by weight). Then, C60PCBM was mixed with the solution in the weight which was three times larger than that of the polymer E. Then, the mixture was filtered with a 1.0 μm Teflon(registered trademark) filter to prepare an applying solution.

A glass substrate with a 150 nm thick ITO film formed by a sputter method was subjected to a surface treatment by an ozone UV treatment. Next, the above applying solution was applied on the substrate by spin coating to form an organic film containing the polymer E and C60PCBM (film thickness: about 80 nm). Then, lithium fluoride was deposited on the organic film in a thickness of 4 nm by a vacuum deposition machine, and then A1 was deposited thereon in a thickness of 100 nm. The obtained organic thin film solar battery had a shape of square measuring 2 mm × 2 mm. The obtained organic thin film solar battery was irradiated with light having a given irradiance using a solar simulator (manufactured by BUNKOUKEIKI Co., Ltd. under the trade name of OTENTO-SUNII:AM1.5G filter, irradiance: 100 mW/cm$^2$). The current and voltage generated were measured and a photoelectric conversion efficiency was determined. The measurement results are shown in Table 5.

Table 5:

| | Conversion efficiency (%) |
|---|---|
| Comparative Example 1 | 0.9 |

Example 14 (Production and Evaluation of Composition and Organic Thin-Film Solar Battery)

In the same manner, except that chlorobenzene was used in place of orthodichlorobenzene in Example 4, a composition ink and an organic thin film solar battery were produced, and then a photoelectric conversion efficiency, a short-circuit current density, an open circuit voltage and a fill factor were determined. The measurement results are shown in Table 6.

Example 15 (Production and Evaluation of Composition and Organic Thin-Film Solar Battery)

In the same manner, except that 1,2,4-trichlorobenzene was used in place of orthodichlorobenzene in Example 4, a composition ink and an organic thin film solar battery were produced, and then a photoelectric conversion efficiency, a short-circuit current density, an open circuit voltage and a fill factor were determined. The measurement results are shown in Table 6.

Example 16 (Production and Evaluation of Composition and Organic Thin-Film Solar Battery)

In the same manner, except that the rotary speed of the spin coater was set at 800 rpm and the film thickness of the organic film was set at 152 nm in Example 4, a composition ink and an organic thin film solar battery were produced, and then a photoelectric conversion efficiency, a short-circuit current density, an open circuit voltage and a fill factor were determined. The measurement results are shown in Table 6.

Example 17 (Production and Evaluation of Composition and Organic Thin-Film Solar Battery)

In the same manner, except that the rotary speed of the spin coater was set at 650 rpm and the film thickness of the organic film was set at 198 nm in Example 4, a composition ink and an organic thin film solar battery were produced, and then a photoelectric conversion efficiency, a short-circuit current density, an open circuit voltage and a fill factor were determined. The measurement results are shown in Table 6.

Example 18 (Production and Evaluation of Composition and Organic Thin-Film Solar Battery)

In the same manner, except that the rotary speed of the spin coater was set at 450 rpm and the film thickness of the organic film was set at 253 nm in Example 4, a composition ink and an organic thin film solar battery were produced, and then a photoelectric conversion efficiency, a short-circuit current density, an open circuit voltage and a fill factor were determined. The measurement results are shown in Table 6.

Table 6:

| | Example 14 | Example 15 | Example 16 | Example 17 | Example 18 |
|---|---|---|---|---|---|
| Short-circuit current density (mA/cm$^2$) | 5.24 | 5.31 | 5.87 | 6.60 | 7.12 |
| Open circuit voltage (V) | 0.85 | 0.85 | 0.84 | 0.85 | 0.85 |
| Fill factor | 0.58 | 0.52 | 0.47 | 0.41 | 0.42 |
| Conversion efficiency. (%) | 2.58 | 2.35 | 2.32 | 2.30 | 2.59 |

Example 19 (Production and Evaluation of Composition and Organic Thin-Film Solar Battery)

In the same manner, except that the rotary speed of the spin coater was set at 850 rpm and the film thickness of the organic film was set at 151 nm in Example 10, a composition ink and an organic thin film solar battery were produced, and then a photoelectric conversion efficiency, a short-circuit current density, an open circuit voltage and a fill factor were determined. The measurement results are shown in Table 7.

Example 20 (Production and Evaluation of Composition and Organic Thin-Film Solar Battery)

In the same manner, except that the rotary speed of the spin coater was set at 700 rpm and the film thickness of the organic film was set at 205 nm in Example 10, a composition ink and an organic thin film solar battery were produced, and then a photoelectric conversion efficiency, a short-circuit current density, an open circuit voltage and a fill factor were determined. The measurement results are shown in Table 7.

Example 21 (Production and Evaluation of Composition and Organic Thin-Film Solar Battery)

In the same manner, except that the rotary speed of the spin coater was set at 500 rpm and the film thickness of the organic film was set at 249 nm in Example 10, a composition ink and an organic thin film solar battery were produced, and then a photoelectric conversion efficiency, a short-circuit current density, an open circuit voltage and a fill factor were determined. The measurement results are shown in Table 7.

Table 7:

| | Example 19 | Example 20 | Example 21 |
|---|---|---|---|
| Short-circuit current density (mA/cm$^2$) | 6.56 | 7.61 | 8.09 |
| Open circuit voltage (V) | 0.84 | 0.84 | 0.84 |
| Fill factor | 0.52 | 0.46 | 0.43 |
| Conversion efficiency (%) | 2.87 | 2.99 | 2.92 |

Example 22 (Production and Evaluation of Composition and Organic Thin-Film Solar Battery)

A glass substrate with a 150 nm thick ITO film formed by a sputter method was subjected to a surface treatment

by an ozone UV treatment using a sputtering method. Next, a solution (concentration: 0.5% by weight) prepared by dissolving polymer A in orthodichlorobenzene was applied on the substrate by spin coating to form an organic film made of the polymer A (film thickness: 30 nm). Then, fullerene C60 was deposited on the organic film in a thickness of 28 nm by a vacuum evaporation system and lithium fluoride was deposited thereon in a thickness of 4 nm by a vacuum evaporation system, and then A1 was deposited thereon in a thickness of 70 nm. The obtained organic thin film solar battery had a shape of square measuring 2 mm × 2 mm. The obtained organic thin film solar battery was irradiated with light having a given irradiance using a solar simulator (manufactured by BUNKOUKEIKI Co., Ltd. under the trade name of OTENTO-SUNII:AM1.5G filter, irradiance: 100 mW/cm$^2$). The current and voltage generated were measured and then a photoelectric conversion efficiency was determined. The measurement results are shown in Table 8. Comparative Example 2 (Production and Evaluation of Composition and Organic Thin-Film Solar Battery)

In the same manner, except that a polymer E was used in place of a polymer A in Example 22, an organic thin film solar battery were produced, and then a photoelectric conversion efficiency, a short-circuit current density, an open circuit voltage and a fill factor were determined. The measurement results are shown in Table 8.

Table 8:

| | Example 22 | Comparative Example 2 |
|---|---|---|
| Short-circuit current density (mA/cm$^2$) | 0.52 | 0.32 |
| Open circuit voltage (V) | 0.83 | 1.01 |
| Fill factor | 0.37 | 0.35 |
| Conversion efficiency (%) | 0.15 | 0.11 |

INDUSTRIAL APPLICABILITY

The photoelectric conversion device produced using the composition of the present invention exhibits high conversion efficiency and is therefore industrially very useful.

## Claims

1. A compound which contains a structure represented by formula (1) and has a light absorption end wavelength of 600 nm or more.

(1)

2. The compound according to claim 1, which further contains a structure different from the structure represented by formula (1).

3. The compound according to claim 2, wherein the structure different from the structure represented by formula (1) is a divalent aromatic group in which two hydrogen atoms have been removed from an aromatic fused ring formed by the condensation of two or more aromatic rings selected from the group consisting of an aromatic 7-membred ring, an aromatic 6-membred ring and an aromatic 5-membered ring and, in the divalent aromatic group, the number of the atoms constituting the aromatic fused ring existing in the shortest path between two atoms having a bond is 3 or 4, including atoms having a bond.

4. The compound according to any one of claims 1 to 3, which has a divalent aromatic group represented by any one of formulas (2) to (4):

(2) (3) (4)

herein, in formulas (2) to (4), $X_1$ to $X_4$ are the same or different and represent a sulfur atom, an oxygen atom, a selenium atom, $-N(R_{10})-$ or $-CR_{11}-CR_{12}-$; $R_{10}$, $R_{11}$ and $R_{12}$ are the same or different and represent a hydrogen atom or a substituent; $R_1$ to $R_6$ are the same or different and represent a hydrogen atom or a substituent, and $R_1$ and $R_2$ may combine to form a cyclic structure and $R_3$ and $R_4$ may combine to form a cyclic structure; $Y_1$ to $Y_4$ are the same or different and represent a nitrogen atom or =CH-.

**5.** The compound according to any one of claims 1 to 4, which is a polymer compound.

**6.** The compound according to claim 5, which has a weight average molecular weight of 10,000 or more.

**7.** A device comprising a first electrode and a second electrode, and an active layer between the first electrode and the second electrode, the active layer containing the compound according to any one of claims 1 to 6.

**8.** A device, wherein at least one layer of the active layer is a layer made of only the compound according to any one of claims 1 to 6.

**9.** A solar battery module comprising the device according to claim 7 or 8.

**10.** An image sensor comprising the device according to claim 7 or 8.

**11.** A method for producing a device comprising a first electrode and a second electrode, and an active layer between the first electrode and the second electrode, the method comprising the steps of applying a solution containing the compound according to any one of claims 1 to 6 and a solvent on the first electrode by an applying method to form the active layer, and forming the second electrode on the active layer.

**12.** The method for producing a device according to claim 11, wherein the applying method is a slit coating method, capillary coating method, a gravure coating method, a microgravure coating method, a bar coating method, a knife coating method, a nozzle coating method, an inkjet coating method or a spin coating method.

**INTERNATIONAL SEARCH REPORT**

International application No.
PCT/JP2010/054735

A. CLASSIFICATION OF SUBJECT MATTER
*C08G61/00*(2006.01)i, *H01L51/30*(2006.01)i, *H01L51/42*(2006.01)i

According to International Patent Classification (IPC) or to both national classification and IPC

B. FIELDS SEARCHED

Minimum documentation searched (classification system followed by classification symbols)
C08G61/00-61/12, H05B33/00-33/28, H01L29/00-31/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JUNG,I.H. et al, Synthesis and electroluminescent properties of fluorene-based copolymers containing electron-withdrawing thiazole derivatives, Journal of Polymer Science, Part A: Polymer Chemistry, 2008, Vol.46, No.21, p.7148-7161 | 1-12 |
| A | PENG,Q. et al, Synthesis and Electroluminescent Properties of Copolymers Based on Fluorene and 2,5-Di(2-hexyloxyphenyl) thiazolothiazole, Macromolecules, 2005, Vol.38, No.17, p.7292-7298 | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 May, 2010 (10.05.10) | 25 May, 2010 (25.05.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/054735

C (Continuation). DOCUMENTS CONSIDERED TO BE RELEVANT

| Category* | Citation of document, with indication, where appropriate, of the relevant passages | Relevant to claim No. |
|---|---|---|
| A | WO 2007/145482 A1 (LG CHEM, LTD.), 21 December 2007 (21.12.2007), claims 1 to 20 & JP 2009-541501 A & EP 2027187 A & KR 10-2007-0119317 A | 1-12 |
| A | WO 2007/125692 A1 (Toyo Ink Manufacturing Co., Ltd.), 08 November 2007 (08.11.2007), claim 1; paragraph [0041] & JP 2007-314757 A & US 2009/0156827 A1 & EP 2025733 A1 & KR 10-2008-0112398 A & CN 101432389 A | 1-12 |
| A | JP 2009-051788 A (Toyo Ink Manufacturing Co., Ltd.), 12 March 2009 (12.03.2009), claims 1 to 5 (Family: none) | 1-12 |
| A | JP 2006-339474 A (Dainippon Printing Co., Ltd.), 14 December 2006 (14.12.2006), claims 1 to 5 & US 2007/0128764 A1 | 1-12 |
| A | OSAKA,I. et al, High-Lamellar Ordering and Amorphous-Like π-Network in Short-Chain Thiazolothiazole-Thiophene Copolymers Lead to High Mobilities, Journal of the American Chemical Society, 2009.01.30, Vol.131, No.7, p.2521-2529 | 1-12 |
| A | JP 2006-077164 A (Sumitomo Chemical Co., Ltd.), 23 March 2006 (23.03.2006), claims 1 to 4; examples (Family: none) | 1-12 |
| P,A | JP 2009-267372 A (Sumitomo Chemical Co., Ltd.), 12 November 2009 (12.11.2009), claims 1 to 6; paragraphs [0044] to [0046] & WO 2009/122956 A1 | 1-12 |

Form PCT/ISA/210 (continuation of second sheet) (July 2009)

INTERNATIONAL SEARCH REPORT

International application No.
PCT/JP2010/054735

Claims 1-12

The inventions in the claims relate to "a compound including the structure represented by formula (1) and having a light absorption edge wavelength of 600 nm or longer". However, compounds having 2,5-bis(3-dodecylthiophen-2-yl)thiazolo[5,4-d]thiazole, 2,1,3-benzothiadiazole, and dioctylfluorene are the only examples of the "compound having the structure represented by formula (1) and having a light absorption edge wavelength of 600 nm or longer" given in the description (examples). It is unclear as to what other structures enable a compound to have a light absorption edge wavelength of 600 nm or longer, even when common technical knowledge at the time of filing is taken into account.

It is also unclear as to in what state (solution state or thin-film state) the compounds were when examined. Furthermore, it is unclear as to what wavelength is meant by the term "light absorption edge wavelength", and no clear definition thereon is given.

Consequently, it is not considered that the inventions in those claims are clearly described. The inventions in those claims are not considered to be sufficiently supported by the description, and are not clearly and sufficiently described in such a degree that a person skilled in the art can practice them.

Incidentally, a search was not made for the inventions which are defined by unclear claims and which are not sufficiently supported by the description and are not clearly and sufficiently disclosed in the description.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9045478 A


### Non-patent literature cited in the description


- *Applied Physics Letters,* 2004, vol. 84 (10), 1653-1655
- *Solar Energy Materials and Solar Cells,* vol. 48, 383-391